# EUROPEAN PATENT SPECIFICATION

(11) **EP 2 882 349 B1**
(45) Date of publication and mention of the grant of the patent: **04.10.2017**
(21) Application number: 13759901.5
(22) Date of filing: 06.08.2013
(51) Int. Cl.: A61B 17/00, A61B 17/064, A61B 17/11, A61B 17/12

(54) **DEVICES FOR ENGAGING TISSUE**
VORRICHTUNGEN ZUM EINGRIFF IN EIN GEWEBE
DISPOSITIFS DE FIXATION À UN TISSU

(30) Priority: 10.08.2012 US 201261682141 P; 06.03.2013 US 201361773442 P; 05.08.2013 US 201313958665
(43) Date of publication of application: 17.06.2015
(60) Divisional application: 17185817.8
(73) Proprietor: W. L. Gore & Associates, Inc., Newark, DE 19711 (US)
(72) Inventor: CULLY, Edward, H., Newark, DE 19711 (US); DUNCAN, Jeffrey, B., Newark, DE 19711 (US); SMITH, Benjamin, A., Newark, DE 19711 (US)
(74) Representative: HGF Limited
(86) International application number: PCT/US2013/053858
(87) International publication number: WO 2014/025825

(56) References cited:
- EP-A2- 1 512 383
- WO-A2-2013/003613
- US-A1- 2003 212 418
- US-A1- 2007 270 943
- US-A1- 2010 140 320
- US-B1- 8 029 529

## Description

### CROSS-REFERENCE TO RELATED APPLICATIONS

This application claims the priority benefit of U.S. Provisional Application No. 61/682,141, filed Aug. 10, 2012, and U.S. Provisional Application No. 61/773,442, filed Mar. 6, 2013.

### FIELD

The present disclosure relates generally to medical devices and, more specifically, to the use of anchors to secure medical devices to tissue in a patient.

### BACKGROUND

Medical devices are frequently used to treat the anatomy of patients. Such devices may be temporarily, semi-permanently, or permanently implanted in the anatomy to provide treatment to the patient. It is important that such devices maintain proper position within the anatomy. Therefore, it is desirable to provide devices, systems and methods for implanting and maintaining position of medical devices within the anatomy of a patient.

US patent application US 2007/270943 (granted US patent US 8932348) relates to a device and method for reducing regurgitation through a mitral valve. The device and method are directed to an anchor portion and deployment of an anchor portion for engagement with the heart wall and an expandable valve portion configured for deployment between the mitral valve leaflets. The valve portion is expandable for preventing regurgitation through the mitral valve while allowing blood to circulate through the heart. The expandable valve portion may include apertures for reducing the stagnation of blood. In a preferred configuration, the device is configured to be delivered in two-stages wherein an anchor portion is first delivered and the valve structure is then coupled to the anchor portion.

European patent EP 1 512 383 relates to a vascular system comprising a balloon catheter and a vascular device. The balloon catheter has an elongated shaft and an expandable balloon. The vascular device is mounted over the expandable balloon and is composed of shape memory material. The vascular device has a collapsed position and a memorized position and is expandable to an expanded position to engage the vessel walls and returnable substantially to the memorized position to bring the walls radially inwardly.

US patent application US2010/140320 (granted US patent US 8500760) relates to a tacking device for engaging tissue, which may be useful for coupling a graft to tissue or facilitating closure of a bodily opening. In one embodiment, the tacking device comprises a main body having proximal and distal ends, and further comprises at least one proximal deployable member and at least one distal deployable member, each having contracted and expanded states. The proximal deployable members extend proximally from the proximal end of the main body, while the distal deployable members extend distally from the distal end of the main body. In one embodiment, a hook member extends from at least one of the proximal deployable members.; In use, the hook member may be engaged, for example, using a loop member coupled to a stylet, thereby facilitating controlled release of the tacking device and allowing repositioning of the tacking device after at least partial deployment of the distal deployable members.

US patent US 8 029 529 relates to a vessel filter including a plurality of struts forming a first collapsible cone expanding radially outward in a proximal direction and a second collapsible cone expanding radially outward in a distal direction. A slidable member is disposed over the plurality of struts to collapse the struts toward a longitudinal axis of the vessel filter. A vessel filter is a device that can be inserted into a blood vessel to capture particles in blood flow.

US 2003/212418 A1 discloses a one piece anastomosis device which is formed of a superelastic or pseudoelastic material which self deforms or self deploys from an insertion configuration to a tissue holding configuration. The device in a deployed state preferably includes an inner tissue penetrating flange which penetrate and retains an everted graft vessel and an outer flange. The self deploying anastomosis device does not rely on a temperature transformation to achieve deployment.

### SUMMARY OF THE INVENTION

The present invention provides an anchor as recited in claim 1.

### BRIEF DESCRIPTION OF THE DRAWINGS

The accompanying drawings are included to provide a further understanding of the disclosure and are incorporated in and constitute a part of this specification, illustrate embodiments and examples of the disclosure, and together with the description, serve to explain the principles of the disclosure, wherein;
FIGS. 1A and 1B illustrate cross sections of delivery systems in accordance with the present disclosure;
FIGS. 2A-2E illustrate perspective views of anchors in accordance with the present disclosure;
Figures 3A and 3B illustrate side views of tissue-penetrating points in accordance with the present disclosure;
Figures 4A and 4B illustrate side views of an anchor in accordance with the present disclosure;
Figures 5A and 5B illustrate a side and perspective view, respectively, of an anchor formation system in accordance with the present disclosure;
Figures 6A-6F illustrate medical devices comprising anchors in accordance with the present disclosure;
Figures 7A and 7B illustrate different stages of deployment of an anchor device in accordance with the present disclosure;
Figure 8 illustrates a flow chart of an anchor deployment method in accordance with the present disclosure;
Figures 9A-9C illustrate various stages of deployment of an anchor in accordance with the present disclosure;
Figures 10A and 10B different various stages of deployment of a plurality of anchors in accordance with the present disclosure;
Figures 11A-11C illustrate various stages of deployment of a medical device comprising an anchor in accordance with the present disclosure;
Figures 12A-12C illustrate various stages of deployment of a device comprising a plurality of anchors in accordance with the present disclosure; and
Figures 13A-13C illustrate a top view and two side views, respectively, of devices comprising a plurality of anchors in accordance with the present disclosure.
Figure 14 illustrates a perspective view of an embodiment comprising a flange element having a cap of biomaterial.
Figures 15A and 15B illustrate a side view and a top view of an example comprising a flange element,

### DETAILED DESCRIPTION OF THE ILLUSTRATED EMBODIMENTS

Persons skilled in the art will readily appreciate that various aspects of the present disclosure can be realized by any number of methods and systems configured to perform the intended functions. Stated differently, other methods and systems can be incorporated herein to perform the intended functions. It should also be noted that the accompanying drawing figures referred to herein are not all drawn to scale, but can be exaggerated to illustrate various aspects of the present disclosure, and in that regard, the drawing figures should not be construed as limiting.

As used herein, the term "elongate element" is generally any element configured for relative axial movement with an endoluminal device delivery element (e.g., a catheter-based endoluminal device delivery element such as a balloon catheter) and includes any longitudinally extending structure with or without a lumen therethrough. Thus, elongate elements include but are not limited to tubes with lumens (e.g., catheters), solid rods, hollow or solid wires (e.g., guidewires), hollow or solid stylets, metal tubes (e.g., hypotubes), polymer tubes, pull cords or tethers, fibers, filaments, electrical conductors, radiopaque elements, radioactive elements and radiographic elements. Elongate elements can be any material and can have any cross-sectional shape including, but not limited to, profiles that are ellipitcal, non-ellipitcal, or random. Typical materials used to construct elongate element, such as catheters, can comprise commonly known materials such as Amorphous Commodity Thermoplastics that include Polymethyl Methacrylate (PMMA or Acrylic), Polystyrene (PS), Acrylonitrile Butadiene Styrene (ABS), Polyvinyl Chloride (PVC), Modified Polyethylene Terephthalate Glycol (PETG), Cellulose Acetate Butyrate (CAB); Semi-Crystalline Commodity Plastics that include Polyethylene (PE), High Density Polyethylene (HDPE), Low Density Polyethylene (LDPE or LLDPE), Polypropylene (PP), Polymethylpentene (PMP); Amorphous Engineering Thermoplastics that include Polycarbonate (PC), Polyphenylene Oxide (PPO), Modified Polyphenylene Oxide (Mod PPO), Polyphenylene Ether (PPE), Modified Polyphenylene Ether (Mod PPE), Thermoplastic Polyurethane (TPU); Semi-Crystalline Engineering Thermoplastics that include Polyamide (PA or Nylon), Polyoxymethylene (POM or Acetal), Polyethylene Terephthalate (PET, Thermoplastic Polyester), Polybutylene Terephthalate (PBT, Thermoplastic Polyester), Ultra High Molecular Weight Polyethylene (UHMW-PE); High Performance Thermoplastics that include Polyimide (PI, Imidized Plastic), Polyamide Imide (PAI, Imidized Plastic), Polybenzimidazole (PBI, Imidized Plastic); Amorphous High Performance Thermoplastics that include Polysulfone (PSU), Polyetherimide (PEI), Polyether Sulfone (PES), Polyaryl Sulfone (PAS); Semi-Crystalline High Performance Thermoplastics that include Polyphenylene Sulfide (PPS), Polyetheretherketone (PEEK); and Semi-Crystalline High Performance Thermoplastics, Fluoropolymers that include Fluorinated Ethylene Propylene (FEP), Ethylene Chlorotrifluroethylene (ECTFE), Ethylene, Ethylene Tetrafluoroethylene (ETFE), Polychlortrifluoroethylene (PCTFE), Polytetrafluoroethylene (PTFE), expanded Polytetrafluoroethylene (ePTFE), Polyvinylidene Fluoride (PVDF), Perfluoroalkoxy (PFA). Other commonly known medical grade materials include elastomeric organosilicon polymers, polyether block amide or thermoplastic copolyether (PEBAX) and metals such as stainless steel and nickel/titanium alloys. The above materials are intended for illustrative purposes only, and not as a limitation on the scope of the present disclosure. Suitable polymeric materials available for use are vast and too numerous to be listed herein and are known to those of ordinary skill in the art.

Medical devices can include, for example, stents, grafts, stent-grafts, filters, valves, occluders, markers, mapping devices, therapeutic agent delivery devices, prostheses, pumps, membranes, patches, meshes, bandages, and other endoluminal and implantable devices are frequently used to treat the anatomy (such as, for example, the vasculature) of patients. Such medical devices can be secured to the anatomy by one or more anchors. In some instances, the anchors are used to hold tissue to tissue as in the case of vascular dissection. In some cases, the anchors can be used to hold medical device to medical device as in the case of modular, multiple component stent-grafts. In some configurations, the anchor(s) can be separate from the medical device. In other configurations, the anchor(s) can be incorporated into and/or integral with the medical device

Anchors in accordance with the present disclosure provide a number of benefits over the prior art. For example, the tips of the anchors that engage tissue in a patient can be positioned in the tissue such that the depth of penetration of the tips is relatively easily controlled. Further, the tips can be everted in a direction and/or position that encourages tissue growth around the tips, subsequently reducing the danger of unintended damage to surrounding tissue caused by the anchor.

In this regard, a delivery system in accordance with the present disclosure can be used to deliver one or more anchors to the anatomy of a patient, wherein the anchor(s) can be incorporated into and/or integral with the medical device, or separate from the medical device, for example a simultaneously, sequentially, or previously implanted medical device.

Further, one or more anchors can used to provide treatment to the anatomy of a patient without an accompanying medical device. For example, one or more anchors can be used to close a wound or otherwise engage tissue to provide a therapeutic or beneficial effect. In other embodiments, one or more anchors can be used to provide a dock for later-deployed medical devices.

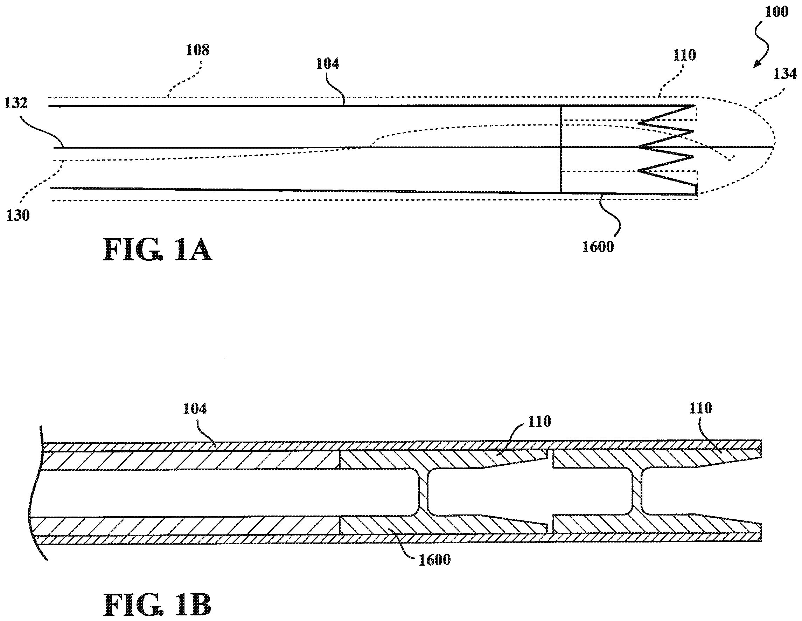

With reference now to Figure 1A, a delivery system 100 in accordance with the present disclosure is illustrated. Delivery system 100 comprises an elongate element 104 capable of delivering at least one anchor 110 to the anatomy of a patient. Delivery systems in accordance with the present disclosure can be configured to deliver at least one anchor 110, and/or both a medical device and at least one anchor 110 in conjunction. For example, as illustrated in Figure 1B, multiple anchors 110 can be consecutively deployed.

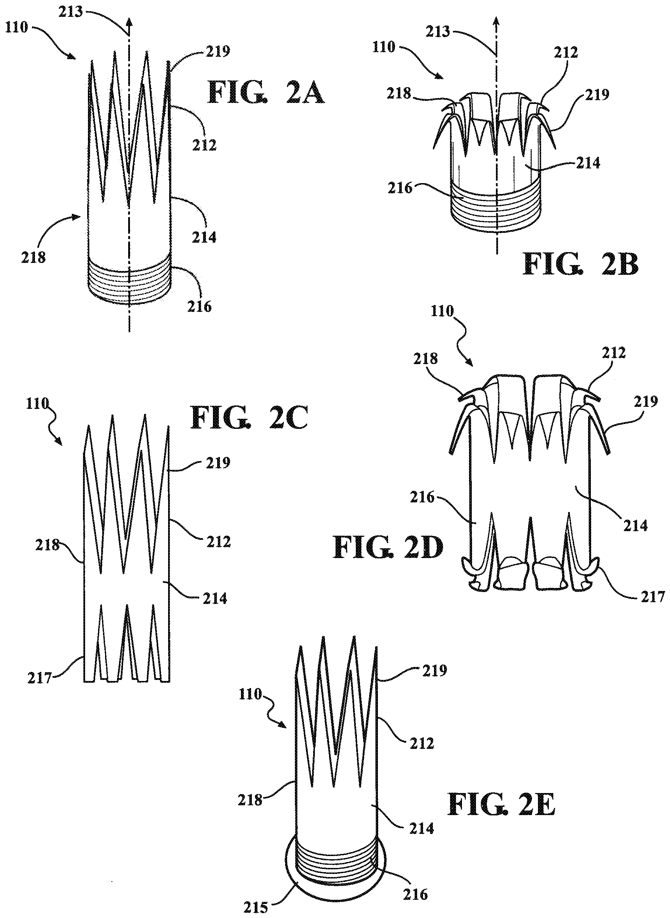

With reference to Figures 2A and 2B, an anchor 110 in accordance with the present disclosure is illustrated. Figure 2A illustrates anchor 110 prior to deployment and Figure 2B illustrates anchor 110 after deployment. Anchor 110 can vary in length, thickness, and diameter. In various embodiments, multiple anchors 110 are utilized to secure a medical device. In such embodiments, anchors 110 can have different length, thickness, and diameter from each other.

In various embodiments, anchor 110 comprises a base portion 214. Base portion 214 can comprise, for example, a generally tubular shape with a central axis 213. Base portion 214 can further comprise an engagement portion 216. In various embodiments, engagement portion 216 is configured to temporarily engage base portion 214 to a delivery system, such as a catheter and/or elongate element. Engagement portion 216 can comprise, for example, a threaded portion located on an outer diameter and/or an inner diameter of base portion 214. For example, engagement portion 216 can comprise of a number of threads along the outer diameter of base portion 214 configured to secure base portion 214 to a complimentarily-threaded section on the inner surface of a tube and/or other member. In other configurations, engagement portion 216 comprises a number of threads along the inner diameter of base portion 214, such that base portion 214 engages with a complimentarily threaded section on the outer surface of an elongate element, tube, and/or other member, such as elongate element 104.

In other embodiments, engagement portion 216 comprises a magnetic portion. For example, engagement portion 216 can comprise a magnetic material coupled to base portion 214. In such configurations, magnetic engagement portion 216 can temporarily couple base portion 214 to a corresponding magnetic portion of a delivery system. Once anchor 110 has been adequately deployed, magnetic engagement portion 216 can disengage from the delivery system. Although described as a threaded portion and a magnetic portion, any engagement portion that can temporarily engage base portion 214 and/or anchor 110 to a delivery system for deployment with the body of a patient is in accordance with the present disclosure.

Base portion 214 can comprise, wholly or in part, for example, a metal or metal alloy with shape-memory properties, such as Nitinol. In other embodiments, base portion 214 comprises a polymeric material capable of bending and/or everting to a predetermined shape or configuration upon deployment. In various embodiments, base portion 214 can comprise a shape that is laser cut from a tube, similar to, for example, a stent. Any material, including various metals and polymers, that is biocompatible and capable of anchoring a medical device to tissue is within the scope of the present disclosure.

In various embodiments, base portion 214 can be compressed or collapsed for delivery into the body of a patient. For example, base portion 214 can be compressed to fit inside a delivery catheter. In such configurations, when deployed, base portion 214 can be expanded, for example, by self-expansion or balloon-assisted expansion, to a larger diameter than the compressed diameter.

Anchor 110 further comprises a plurality of anchor arms 212. In various embodiments, anchor arms 212 are positioned circumferentially on base portion 214. Anchor arms 212 of anchor 110 can comprise a shaft portion 218. Prior to deployment of anchor 110, shaft portion 218 is substantially parallel to central axis 213 of base portion 214.

In various embodiments, shaft portion 218 can be slit or lanced, such that shaft portion 218 can bend and/or evert during deployment of anchor 110. In such configurations, shaft portion 218 can evert during deployment such that anchor arms 212 bend up to, for example, 180 degrees, 360 degrees, or more from their original orientation. Such eversion can resemble a mushroom-style deformation, as each anchor arm 212 is bent to a similar degree and shape. Shaft portion 218 everts such that each anchor arm 212 is substantially parallel to central axis 213.

Shaft portion 218 can comprise, for example, a material that allows shaft portion 218 to evert and/or bend in a predetermined manner. In various embodiments, shaft portion 218 comprises a metal or metal alloy with shape-memory properties, such as Nitinol. In other embodiments, shaft portion 218 comprises a polymeric material, such as a shape-memory polymer taught in U.S. Patent 7,498,385 to Swetlin et al, which is capable of bending and/or everting to a predetermined shape or configuration upon deployment. Typically, all components of anchor 110, including base portion 214, anchor arms 212, and shaft portion 218 are comprised of the same material. Any material, including various metals and polymers, that is biocompatible and capable of anchoring a medical device to tissue is within the scope of the present disclosure. Materials that can be appropriate include, but are not limited to full hard 316 stainless steel or L605 and Eligiloy and other materials which have self-deploying characteristics.

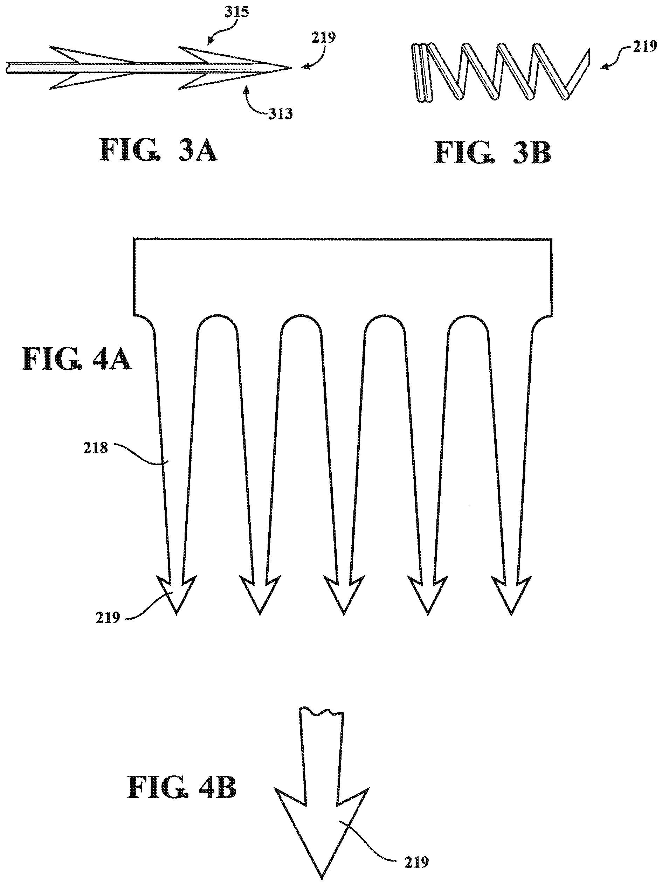

Anchor arms 212 further comprise a tissue-penetrating point 219. In various embodiments, tissue-penetrating point 219 is located at the end of shaft portion 218. Tissue-penetrating point 219 can comprise a shape capable of penetrating tissue and securing anchor 110 to the anatomy of the patient. As illustrated in Figure 2A, tissue-penetrating point 219 can be, for example, substantially arrow-shaped. With reference to Figures 4A and 4B, multiple shaft portions 218, each equipped with a substantially arrow-shaped tissue-penetrating point 219 are illustrated.

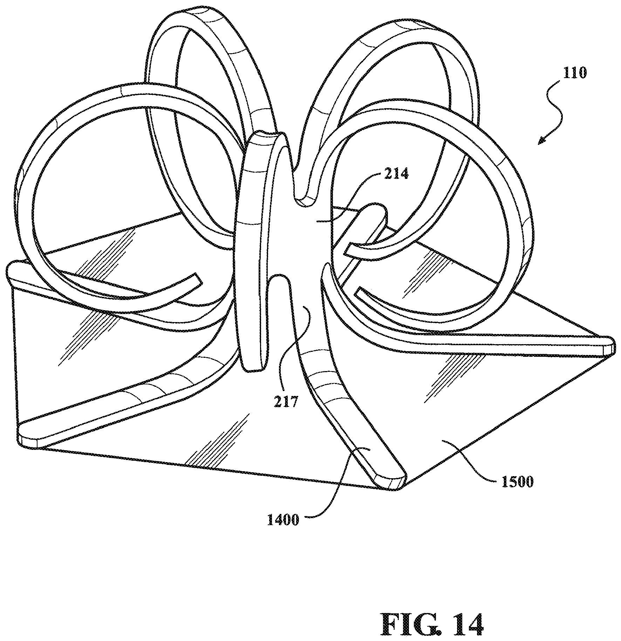

With reference to Figures 2C and 2D, an anchor 110 in accordance with the present disclosure can further comprise at least one flange element 217. In such configurations, flange elements 217 can evert after anchor arms 212 have everted, such that a medical device and/or the tissue engaged by anchor arms 212, shaft portion 218, and tissue-penetrating point 219 is sandwiched between anchor arms 212 and at least one flange element 217. In these embodiments, flange elements 217 can maintain proximal positioning between a medical device and the tissue engaged by anchor 110. In various embodiments, including the embodiment of Figure 14, the flange element 217 has one or more flange element arms 1400 having a portion of the one or more flange element arms substantially everting to a position approximately 90 degrees from the central axis of the base portion 214 of the anchor 110. The flange element 217 is atraumatic. As used herein, "atraumatic" refers to the flange element 217 and the flange element arms 1400 are designed to minimize or avoid penetration of tissue or a medical device or to avoid causing damage to tissue.

In various embodiments, and with reference to Figure 3A, tissue-penetrating point 219 can comprise a barb 313 with one or more fins 315. With reference to Figure 3B, tissue-penetrating point 219 can comprise a corkscrew-like configuration. Although described in a number of specific configurations, such as, for example, including arrow-shaped, barbed, and corkscrew-like, any configuration of tissue-penetrating point 219 that suitably engages with and secures a medical device to tissue is within the scope of the present disclosure. In various embodiments, the anchors 110 or one or more portions of the anchors such as the base portion 214, engagement portion 216, one or more anchor arms 212, one or more shaft portions 218, one or more tissue-penetrating points 219, one or more flange elements 217, and or one or more barbs 313 or fins 315 or combinations of any of the foregoing can be partially, substantially or wholly covered in a porous or fibrous biomaterial. The porous or fibrous biomaterial assists with the integration of the anchor 110 with the surrounding tissue. Biomaterials provide an open structure on the surface of the anchor 110 that is sufficiently large for cells to readily penetrate and promote ingrowth of both collagenous and vascular tissues for example. Porous structures for implantable devices sufficiently large to allow ingrowth and attachment of tissue can be achieved through a variety of means. Various technologies are able to deliver tailored open-celled structures with various pore sizes to fit the particular cell ingrowth applications. Such materials include fluorinated polymers and copolymers; expanded fluorinated polymers and copolymers; and woven, non-woven, extruded or the like fibers including PGA:TMC copolymer (polyglycolic acid:trimethylenecarbonate copolymer) fibers. Any combination of these porous or fibrous biomaterials can be utilized in various embodiments. In various embodiments, expanded polytetrafluoroethylene (ePTFE) is used as a covering on one or more portions of the anchors 110. Various means for attaching the biomaterials can be utilized such as attaching with fluorinated ethylene propylene (FEP). Figure 14 illustrates a perspective view of an embodiment of an anchor 110 comprising a flange element 217 with flange element arms 1400 that are indexed to minimize interaction between the anchor arms 212 and the flange element arms 1400. Figure 14 also illustrates an embodiment with a fluorinated polymer, specifically an expanded polytetrafluoroethylene (ePTFE), biomaterial attached to the flange element as a cap 1500. The cap promotes tissue ingrowth and can be used to encase the flange element 217 of the anchor 110, thereby rendering it atraumatic.

In some embodiments, the anchor 110 or portions thereof can be covered with one or more bioactive agents to initiate a bio-response. Examples of such bioactive agents include antimicrobials, PGA:TMC , and anticoagulants such as Heparin. It should be noted that combinations of such bioactive agents can be applied even within the same anchor 110. For instance, in the case of an anchor 110 used to tack a vascular graft to the aortic wall, the anchor arms 212 can be coated with a substance which is known to generate a tissue healing response, while the flange elements 217 (and cap 1500) can be treated with Heparin, to mitigate clotting in the blood stream.

In various embodiments, the anchor 110 or any combination of portions thereof can be surface treated, for example sand blasted, coated by spraying or dipping for example to coat with a bioactive agent, and covered with a porous or fibrous biomaterial for example to initiate various desired bio-responses such as tissue ingrowth or anticoagulant responses,

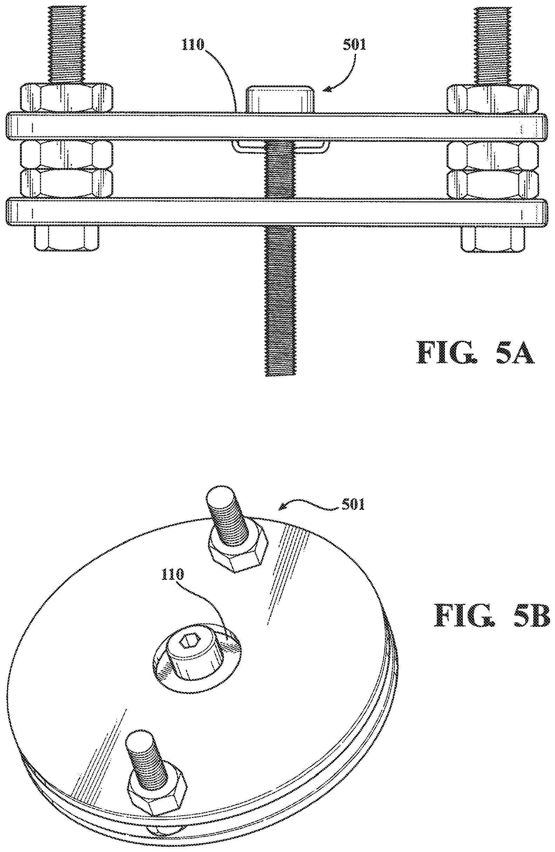

With reference to Figures 5A and 5B, anchors 110 can be formed by placing a laser-cut tube comprising, for example, Nitinol, in a form configured to conform the tube into the desired deployment configuration of anchor 110. For example, a tube is placed into form 501. Form 501 everts anchor arms 212 of the tube, creating the deployed configuration of anchor 110. In various examples, form 501 and anchor 110 are placed in an oven to heat treat and set the deployed configuration of anchor 110.

In examples in which anchor 110 is produced by heat treating, operating conditions of the heat treating process can be varied to produce different characteristics in anchor 110. For example, heat treating Nitinol at a relatively low temperature can produce a softer material, which may produce an anchor 110 that is easier to remove from tissue in the body of a patient. Treating Nitinol at a relatively higher temperature can produce a harder material, which can produce an anchor 110 that has improved grip and engagement with tissue of the patient.

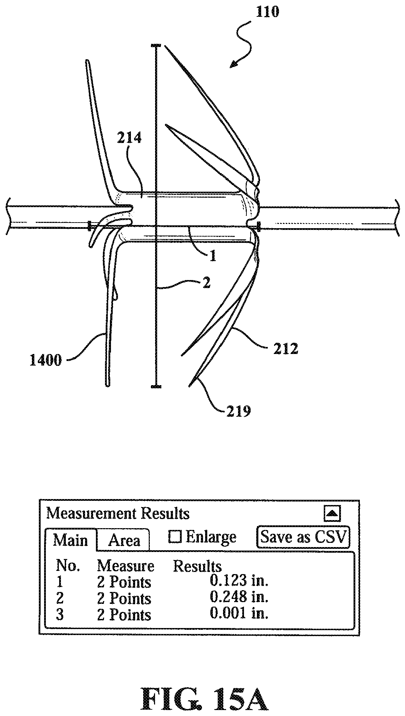

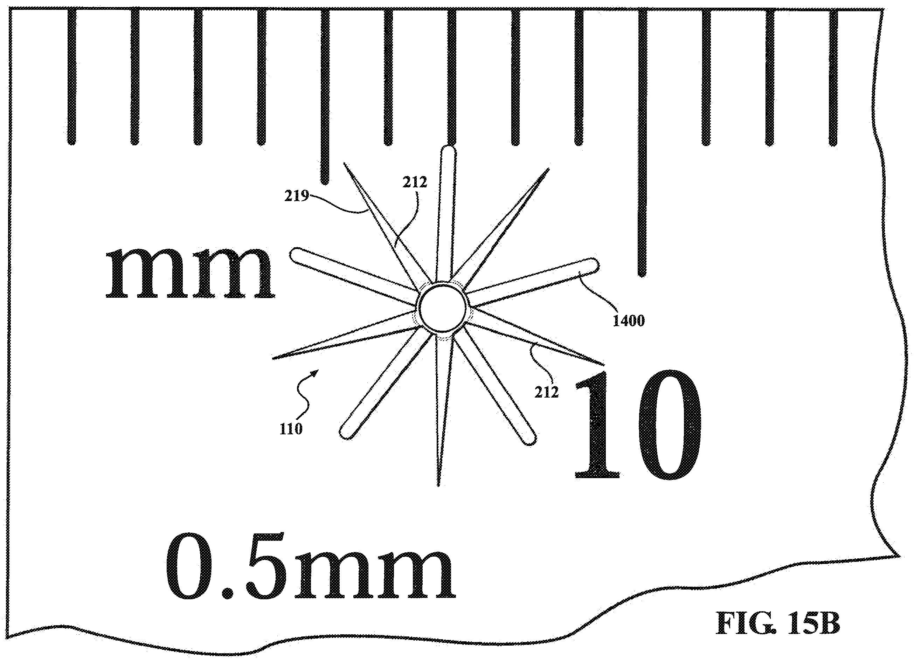

In an example, with reference to FIGS. 15A and 15B, a laser-cut tube of Nitinol having an outer diameter of about 0.76mm (0.030inches) was heat treated to result in an anchor 110 that can be delivered via lumen of a 4 fr catheter, elongate element. The anchor of FIGS. 15A and 15B has anchor arms 212 with tissue penetrating points 219 and atraumatic flange element arms 1400 with blunt ends as shown in FIG. 15B. The anchor of FIGS. 15A and 15B when deployed has a length (shown as 1) of about 3.05mm (0.12 inches). and a width (shown as 2) of about 6.35 mm (0.25 inches). With reference to FIG. 15B, the anchor arms 212 and the flange element arms 1400 are indexed. Indexing avoids interference between the deploying anchor arms 212 and the flange element arms 1400. Both deploy away from the central axis of the base portion 214.

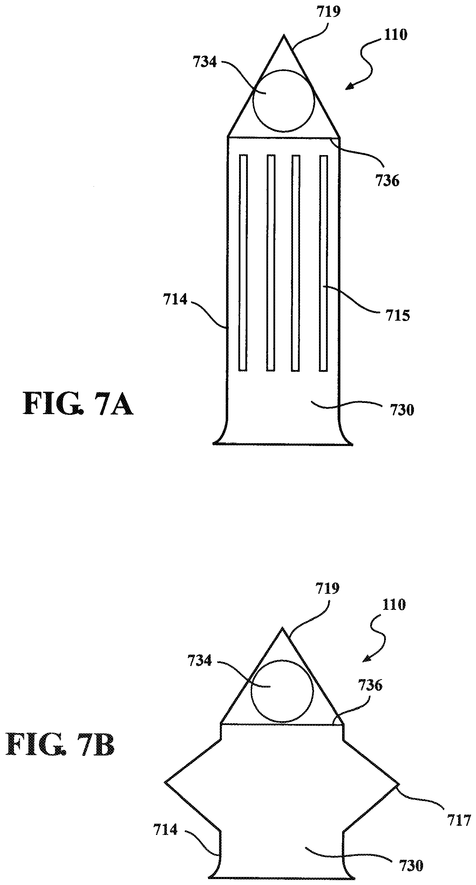

In various examples, with reference to FIG. 7A, anchor 110 comprises a substantially cylindrical body 714 and a tissue-penetrating point 719. Cylindrical body 714 can further comprise a plurality of slots 715.

Anchor 110 can further comprise a tip 734, activation wire 730, and retention element 736. In such configurations, tip 734 can comprise a ball affixed (by, for example, welding) to activation wire 730. When tension is applied to tip 734 by activation wire 730, tip 734 is drawn towards the base of anchor 110. Because tip 734 is held in place inside of anchor 110 by retention element 736, the force applied to tip 734 causes cylindrical body 714 to partially collapse.

As illustrated in FIG. 7B, partial collapse of cylindrical body 714 can cause the material between slots 715 to expand radially, creating a number of tissue-engaging points 717. Once sufficient engagement between tissue and anchor 110 is achieved, anchor 110 can be disengaged from elongate element 104. In various examples, retention element 736 can comprise, for example, an element that disengages when sufficient force is applied. In such configurations, sufficient force to disengage retention element 736 is higher than the force required to partially collapse cylindrical body 714. In other examples, retention element 736 comprises member having a hole, such that as cylindrical body 714 partially collapses, the hole of retention element 736 increases in diameter. After sufficient collapsing of cylindrical body 714, tip 734 can pass through retention element 738 and be removed from the patient.

As discussed previously, anchors 110 can be utilized and/or deployed independently from medical devices to secure medical devices to the anatomy of a patient, for example a simultaneously, sequentially or previously implanted medical devices. For example, with reference to Figure 2E, one or more anchors 110 can be utilized to secure a properly positioned graft member to the wall of a vessel, such as the aorta. In such configurations, the graft member can be positioned within the vessel, and anchors 110 can be deployed such that the end of the anchor passes through the graft member and engages the vessel. Flared segment 215, which can comprise a portion having a larger diameter than the diameter of base portion 214, such that flared segment 215 can assist in maintaining proper positioning of the graft member relative to the vessel.

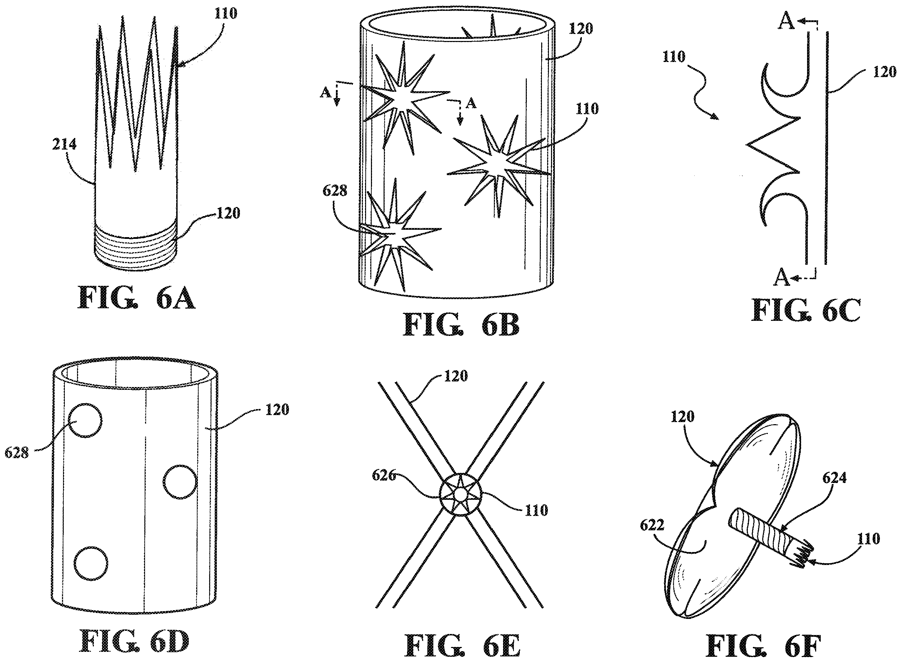

Figure 6D illustrates another embodiment in which an anchor or multiple anchors 110 can be used to secure medical device 120, such as a stent or stent graft, to the anatomy of a patient. In such embodiments, medical device 120 is a stent or stent graft with one or more holes 628. Holes 628 can be configured, for example, to allow an anchor 110, as illustrated in Figure 2E, to pass through the stent or stent graft and engage the anatomy of the patient. In such configurations, flared segment 215 can comprise a larger diameter or cross sectional profile than holes 628, such that anchor 110 is capable of securing medical device 120 through holes 628. Such engagement can retain the proper positioning of medical device 120 within the anatomy.

In other embodiments, anchor 110 can be incorporated into and/or integral with medical device 120. Figures 6A, 6B, 6C, 6E, and 6F illustrate anchors 110 that are incorporated into and/or integral with medical devices 120. For example, as illustrated in Figure 6A, a medical device 120 can comprise a drug-eluting button that can be incorporated into base portion 214 of anchor 110. In other embodiments, medical device 120 can comprise an intercardiac device, such as a device designed to transmit electrical energy to tissue of the heart.

In various examples, for example as illustrated in Figures 6B and 6C, one or more anchors 110 can be incorporated into medical device 120. For example, medical device 120 can comprise a metal tube with one or more holes 628. The shape of holes 628 can comprise one or more points 629. In such configurations, points 629 comprise anchors 110. Medical device 120 can be deployed into a vessel, and once properly positioned, anchors 110 can be deployed such that points 629 evert, as illustrated in 6C, and engage with the vessel wall.

In various embodiments, for example as illustrated in Figures 6E, anchors 110 can connect directly to and deploy concurrently with medical device 120. For example, medical device 120 can comprise a stent that is constructed from metal rings joined together at apices. In such embodiments, one or more anchors 110 are positioned at one or more of the apices 626 of the stent of medical device 120. When deployed, anchors 110 secure the stent of medical device 120 to the anatomy of the patient. In such embodiments, anchors 110 can remain connected to medical device 120 after deployment, maintaining engagement between medical device 120 and the anatomy of the patient. Although anchors 110 are described as located at apices 626, anchors can be located anywhere along the stent of medical device 120.

With reference to Figure 6F, medical device 120 can comprise a modified occluding device, such as a single-disk occluding device for use in reducing the volume of a left atrial appendage of the heart. In such embodiments, one of the two disks can be removed and replaced with an anchor 110. A connecting element 624 can connect an occluding disk 622 to anchor 110. Anchor 110 can then properly position the remaining occluding disk 622 relative to the left atrial appendage, obviating the need for the second disk. Although described in connection with a single-disk occluding device, medical device 120 can be any device that can combine with or incorporate into and/or be formed integral with anchor 110 and deployed to the anatomy of a patient.

With reference back to Figure 1A, delivery system 100 can further comprise a sheath 108, such as, for example, a delivery sheath. Sheath 108 can be configured to surround anchor or anchors 110, a medical device, or both anchors 110 and a medical device. In various embodiments, sheath 108 can assist in the deployment of anchors 110 and/or a medical device. In various embodiments, the portion 1600 of the elongate element 104, catheter or sheath, as shown in Figures 1 A and 1 B, that houses the anchor or anchors when loaded and during unloading can be comprised of various materials or composites to minimize interaction between the anchors and the inner diameter of the elongate element. These materials include, but are not limited to, metals or alloys such as Nitinol and stainless steel, high durometer plastics/polymers, radiopaque cuffs/bands, or any similar biocompatible material. Alternatively, the inner diameter of that portion of the elongate element can be coated or have an insert of such materials to minimize the interaction of the inner diameter of that portion with the anchors. Use of these materials in the loading and delivery portion of the elongate element or catheter can minimize particulation and improve delivery of the anchors.

In various embodiments, delivery system 100 further comprises an activation wire 132. Optionally, delivery system can further comprise a tip 134. As will be discussed later in greater detail, activation wire 132 and/or tip 134 can assist in the deployment of anchors 110 and/or medical device 120.

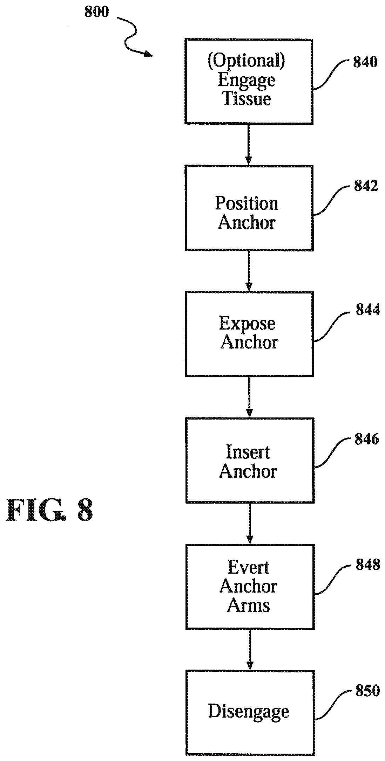

With reference to Figure 8, an anchor deployment method 800 in accordance with the present disclosure is illustrated. In various examples, delivery system 100 can be used to deliver and deploy one or more anchors 110 according to anchor deployment method 800.

Anchor deployment method 800 comprises an optional engage tissue step 840. For example, engage tissue step 840 can comprise using a fixation wire 130 to temporarily engage the tissue of the anatomy by entering the tissue. Fixation wire 130 can comprise a point that is capable of piercing and embedding in tissue. Once embedded, the point of fixation wire 130 can allow the operator to position elongate element 104, sheath 108, and anchor 110 for deployment. Although described in connection with fixation wire 130, any device or tool that allows for temporary engagement of tissue and positioning of sheath 108, and/or anchor 110 is within the scope of the present disclosure.

In various examples, anchor deployment method 800 further comprises a position anchor step 842. Position anchor step 842 can comprise elongate element 104 and sheath 108 such that anchor 110 is located in proximity to the desired deployment location in the anatomy of the patient. For example, position anchor step 842 can comprise using fixation wire 130 to assist in directing elongate element 104, sheath 108, and anchor 110 to the proper position for deployment of anchor 110.

Anchor deployment method 800 further comprises an expose anchor step 844. In various examples, expose anchor step 844 can comprise preparing anchor 110 for insertion into the tissue of the anatomy. For example, expose anchor step 844 can comprise withdrawing sheath 108 from anchor 110 and exposing at least a portion of anchor 110.

In various examples, anchor deployment method 800 further comprises an insert anchor step 846. Insert anchor step 846 can comprise, for example, using delivery system 100 to insert anchor arms 212 of anchor 110 into the tissue of the anatomy. In such configurations, tissue-penetrating point 219 of anchor arm 212 can allow anchor arms 212 to effectively penetrate and engage the tissue at the desired location within the anatomy.

Anchor deployment method 800 further comprises an evert anchor arms step 848. In various examples, evert anchor arms step 848 can comprise bending and/or everting shaft portion 218 of anchor arms 212. For example, shaft portions 218 of anchor arms 212 can be bent such that anchor arms 212 are in a mushroom-shaped configuration, and at least a segment of shaft portion 218 is substantially parallel to central axis 213. In various embodiments, anchor arms 212 comprise a shape memory metal alloy, such as Nitinol, which has been pre-set to the desired everted configuration. As anchor 110 is inserted into the tissue of the patient, anchor arms 212 return to the everted pre-set configuration.

In various examples, evert anchor arms step 848 comprises bending and/or everting anchor arms 212 using tip 134 of delivery system 100. For example, tip 134 can be located concentrically to and extend past tissue-penetrating points 219 of anchor arms 212. Tip 134 can comprise a portion that is larger than the diameter of anchor 110. When anchor 110 is engaged in the tissue at the desired location in the anatomy, tip 134 can be retracted, exerting pressure on and causing plastic deformation of anchor arms 212, thereby bending and/or everting shaft portions 218. However, any manner of bending and/or everting anchor arms 212 to cause a sufficiently strong and secure engagement between anchor 110 and the desired tissue is within the scope of the present disclosure.

Anchor deployment method 800 further comprises a disengage step 850. Disengage step 850 can comprise, for example, separating anchor 110 from delivery system 100. In various examples, anchor 110 is uncoupled from elongate element 104 by rotating elongate element 104 until the threads of anchor 110 and elongate element 104 disengage from each other. In other examples, anchor 110 can be coupled to elongate element 104 by other methods, such as, for example, a tension fit. In such configurations, disengage step 850 comprises uncoupling anchor 110 and elongate element 104 by the appropriate method.

Disengage step 850 can further comprise removing any temporary engagement device or tool from the tissue of the anatomy. For example, in examples in which fixation wire 130 is used in engage tissue step 840, disengage step 850 can comprise removing fixation wire 130 from the tissue of the anatomy.

Anchor deployment method 800 can be used in conjunction with the delivery of a variety of different medical devices 120. For example, as described in relation to anchor 110 of Figure 2A, anchor deployment method 800 can be used to deliver an integrated anchor 110 and medical device 120. In such examples, multiple integrated anchors 110 and medical devices 120 can be delivered to different locations within the anatomy using the same delivery system 100.

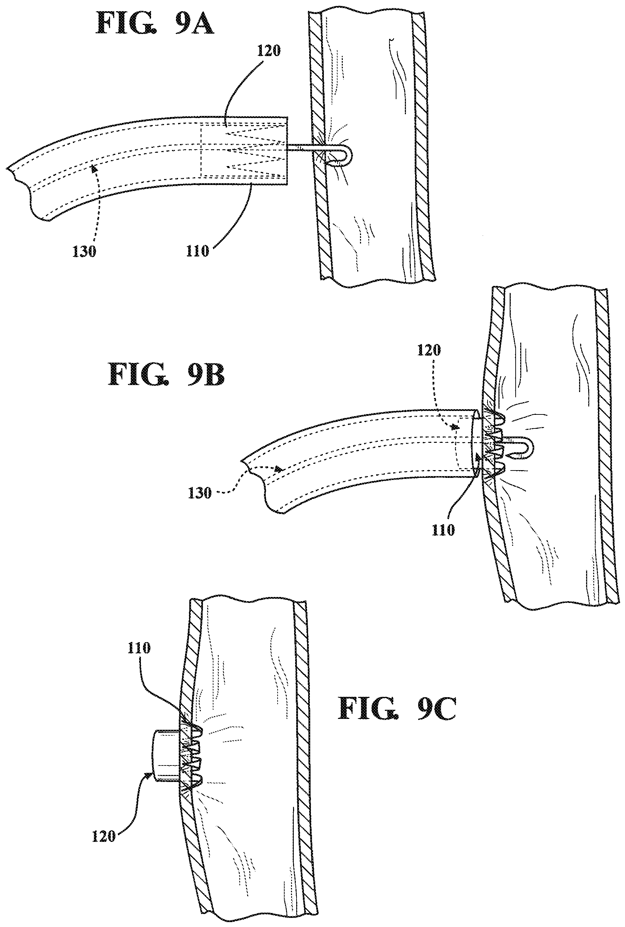

For example, with reference to Figures 9A-9C, the deployment of a medical device 120 integrated with anchor 110 is illustrated. For example, Figure 9A illustrates anchor 110, and fixation wire 130 during engage tissue step 840 of anchor deployment method 800. Figure 9B illustrates such a system during an evert anchor arms step 848 of anchor deployment method 800. Figure 9C illustrates anchor 110, and fixation wire 130 after the completion of disengage step 850 of anchor deployment method 800.

In various embodiments, a plurality of anchors 110 can be deployed to secure medical device 120 to the anatomy of the patient. For example, as described in relation to Figure 6B, medical device 120 can comprise holes 628 configured to allow anchors 110 to pass through from the inside of medical device 120 and contact the tissue of the anatomy. In such configurations, anchors 110 can be deployed individually, either in sequence or simultaneously.

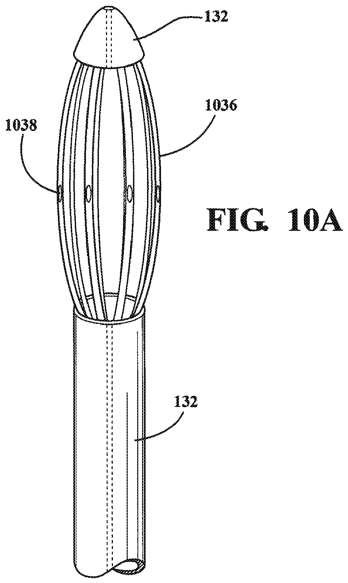

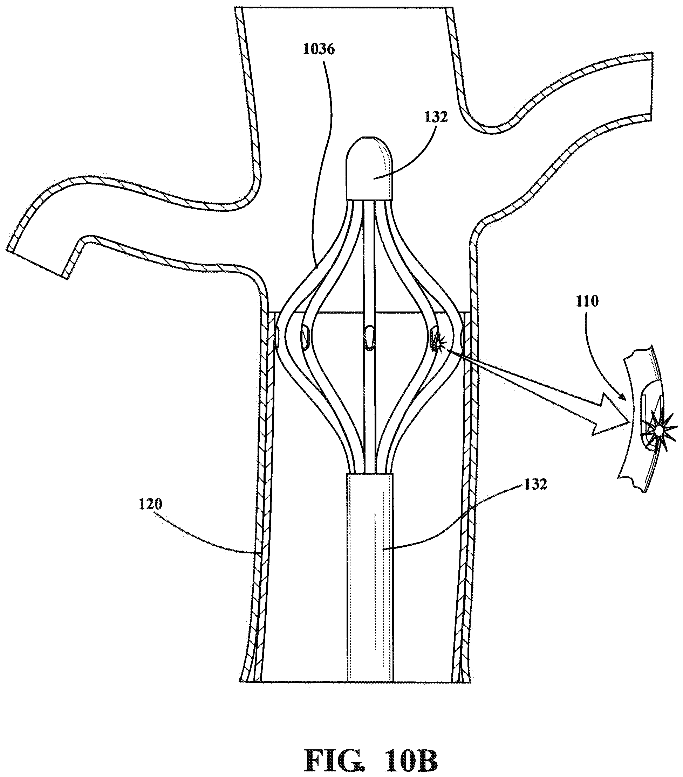

For example, as illustrated in Figures 10A and 10B, a number of anchors 110 can be deployed simultaneously to secure medical device 120 to the tissue of the anatomy. Figure 10A illustrates a delivery system comprising a series of hypotubes 1036, each comprising a trap door 1038. Each of a plurality of anchors 110 is stored in hypotubes 1036. In various embodiments, each trap door 1038 corresponds to a position along medical device 120 to be secured to the anatomy of the patient. For example, the positions of trap doors 1038 can correspond with locations of holes 628 of medical device 120 illustrated in Figure 6B.

As illustrated in Figure 10B, activation wire 132 can be configured such that when tension is applied to it, hypotubes 1036 compress longitudinally and expand perpendicularly. Further, tension applied to activation wire 132 exposes anchors 110 to the anatomy of the patient. In such embodiments, applying sufficient tension to activation wire 132 causes anchors 110 to engage with the tissue of the anatomy simultaneously. After anchors 110 have sufficiently engaged the anatomy, tension can be released from activation wire 132, and anchors 110 thereby disengaged.

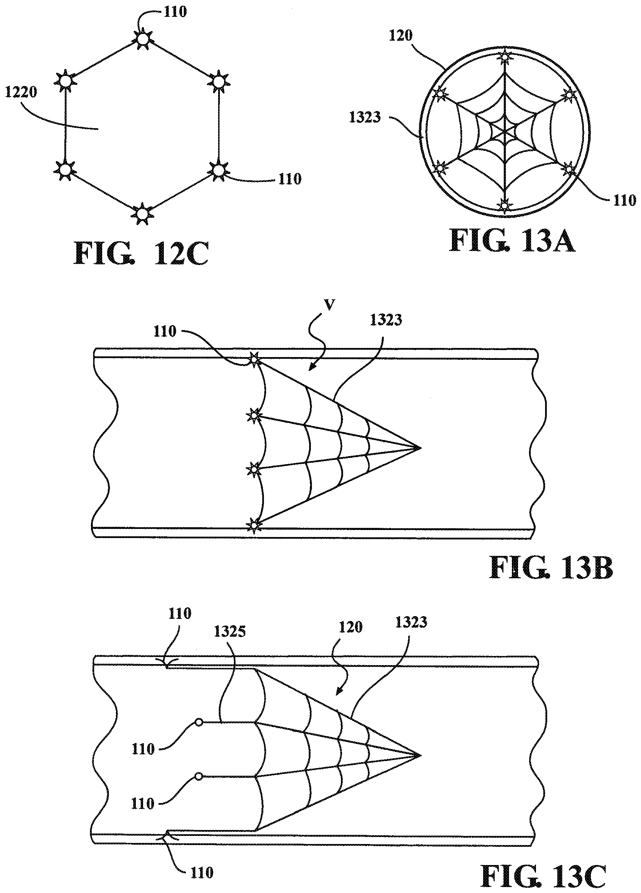

With reference to Figures 13A-13C, in various embodiments, medical device 120 can comprise a net 1323 and one or more anchors 110. As illustrated in Figure 13A, net 1323 can comprise a substantially round cross section having a plurality of anchors 110 disposed around the perimeter of net 1323. When deployed in the vasculature of a patient, as illustrated in Figures 13B and 13C, net 1323 can have a substantially cone-shaped profile that allows it to trap and retain debris inside of a vessel. Such a configuration allows for the temporary, semi-permanent, or permanent installation of net 1323 in a particular vessel.

In various embodiments, net 1323 comprises a plurality of biocompatible, polymeric threads. For example, net 1323 can comprise a plurality of ePTFE threads joined to form a substantially round cross section and a substantially cone-shaped profile. However, net 1323 can comprise any material that can trap and retain debris in the vasculature of a patient.

Net 1323 and anchors 110 can be deployed as illustrated in Figures 10A and 10B. With momentary reference to these figures, anchors 110 are simultaneously deployed through hypotubes 1038, engaging in the vasculature of the patient and providing anchoring for net 1323. Benefits of such deployment of net 1323 include allowing for bi-directional deployment and retrievability, reduced vessel wall penetration due to reduced outward radial force against the vessel during deployment, low or no tilting of net 1323 relative to the vessel wall, and limited or no fracture of net 1323. While the illustrated deployment method provides a number of benefits, any deployment method that successfully implants, temporarily, semi-permanently, or permanently, net 1323 and anchors 110 within the vasculature of a patient is within the scope of the present disclosure.

In other embodiments, as illustrated in Figure 13C, medical device 120 comprises net 1323, anchors 110, and a plurality of tethers 1325. In such configurations, one or more tethers 1325 extend from a location along the perimeter of net 1323. Each tether 1325 engages with an anchor 110. In various embodiments, tethers 1325 are substantially the same length as one another. In other embodiments, tethers 1325 comprise at least two different lengths, such that at least two tethers 1325 are not the same length as one another. Although described in connection with tethers, any manner of utilizing anchors 110 to affix net 1323 to the vasculature of a patient is within the scope of the present disclosure.

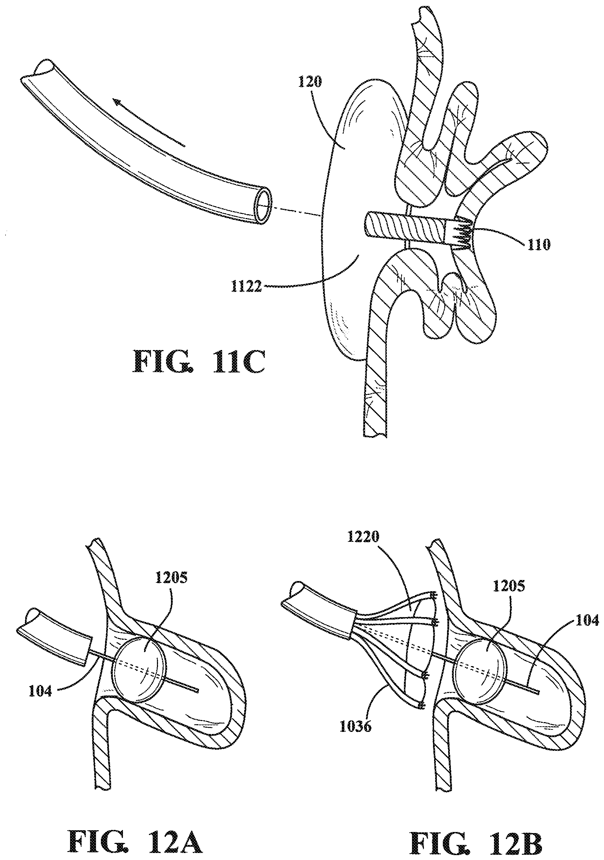

In other embodiments, multiple anchors 110 can be deployed sequentially or simultaneously to secure a device, such as an occluder or a patch, to seal off, for example, a vessel, portion of a vessel, or left atrial appendage. For example, with reference to Figures 12A-12C, patch 1220 can be secured to the tissue of a patient by multiple anchors 110. As illustrated in Figure 12A, elongate element 104, and balloon 1205 can interface with a treatment area, such as, for example, a left atrial appendage. Balloon 1205 can assist in positioning elongate element 104 relative to the left atrial appendage. As illustrated in Figure 12B, a number of hypotubes 1036 can be used to deliver multiple anchors 110. In various embodiments, each hypotube 1036 can deliver one anchor 110 to the tissue of the treatment area.

In various embodiments, multiple anchors 110 can simultaneously engage with and secure patch 1220 to the tissue of the heart surrounding the left atrial appendage. As illustrated in Figure 12C, patch 1220 can be secured at numerous points along the body, including the perimeter, of patch 1220, thereby sealing off the left atrial appendage.

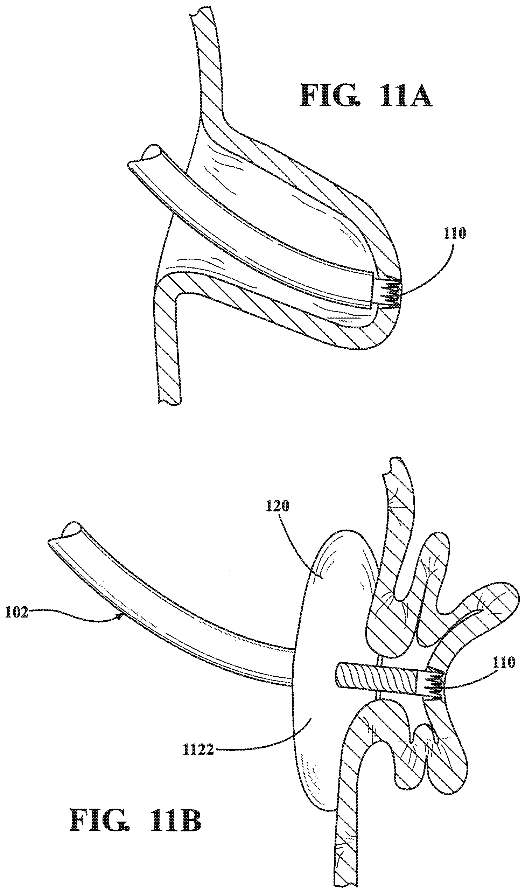

In other embodiments, as described in relation to Figure 6C, one or more of a combination medical device 120 and anchor 110 can be deployed. For example, as illustrated in Figures 11A-11C, medical device 120 can comprise an occluding disk 1122 coupled to at least one anchor 110. In such configurations, one or more anchors 110 assist in positioning and engagement of medical device 120.

Figure 11A illustrates a delivery system used to position and deploy anchor to a desired treatment area, such as, for example, the left atrial appendage of a patient. Anchors 110 can be deployed such that the anchor engages a portion of the left atrial appendage. Once anchor 110 has engaged the left atrial appendage, the delivery system can be withdrawn, which reduces the volume of the left atrial appendage.

In various embodiments, as illustrated in Figure 11B, medical device 120 can be deployed from the delivery system after the left atrial appendage has been properly engaged and the volume sufficiently reduced. Subsequently, as illustrated in Figure 11C, medical device 120 can be released from the delivery system, allowing occluding disk 1122 to engage with the left atrial wall to maintain the reduced volume of and prevent blood flow into the left atrial appendage.

In various embodiments, anchors 110 can be removed from the tissue of a patient. For example, elongate element 104 can be used to remove one or more anchors 110. In such configurations, elongate element 104 can be reengaged with anchor 110 by, for example, coupling the threaded portion of elongate element 104 with the complimentarily threaded portion of anchor 110. Elongate element 104 can then be retracted, causing anchor arms 212 to disengage with the tissue and allowing for the removal of anchor 110.

Although various particular embodiments and examples are particularly described herein, any combination of anchor 110 and medical device 120 that provides a desired treatment to a patient is within the scope of the present disclosure. Further, any order of deployment that provides suitable positioning and engagement of medical device 120 with the anatomy of the patient is within the scope of the present disclosure. Specifically, one or more anchors 110 and one or more medical devices 120 can be deployed using a single or multiple delivery systems 100, in any order of deployment that achieves the desired result. For example, medical devices 120 can be deployed before, during, or after the deployment of one or more anchors 110, and vice versa.

Whilst specific embodiments of the present invention have been described above, it will be appreciated that departures from the described embodiments may still fall within the scope of the present invention.

## Claims

1. An anchor (110), comprising;
a base portion (214) having a generally tubular shape defining a lumen having a central axis (213), wherein the base portion (214) comprises an engagement portion (216) configured to temporarily engage said base portion (214) to a delivery system; and
a plurality of shape memory anchor arms (212) coupled to the base portion (214) such that the plurality of shape memory anchor arms (212) are configured to be substantially parallel to a central axis (213) of the base portion (214) prior to deployment, each of the plurality of shape memory anchor arms (212)
being comprised of a tissue-penetrating point (219) coupled to and supported by a shaft portion (218) which is configured for eversion during deployment, and
wherein each of the shaft portions is configured to evert away from the central axis (213) of the base portion during deployment; and
at least one flange element (217) comprising one or more flange element arms (1400) having a portion of the one or more flange element arms (1400) substantially everting to a position approximately 90 degrees from the central axis (213) of the base portion (214) of the anchor (110); and
wherein the at least one flange element (217) is atraumatic;
**characterised by**
a fluorinate polymer biomaterial attached to the flange element (217) as a cap (1500).

2. The anchor (110) of claim 1, wherein the anchor (110) is deployable along an elongate element (104).

3. The anchor (110) of claim 2, wherein the engagement portion (216) is a threaded portion engageable- by the elongate element (104) to retract the plurality of shape memory anchor arms (212) from a tissue.

4. The anchor (110) of claim 3, wherein the threaded portion is located along an inner diameter of the base portion (214).

5. The anchor (110) of claim 1, wherein the engagement portion (216) comprises a magnetic portion engageable with a corresponding magnetic portion of a delivery system to deploy the anchor (110).

6. The anchor (110) of claim 1, wherein the tissue-penetrating point (219) of each of the plurality of shape memory anchor arms (212) points substantially parallel to the central axis (213) of the base portion (214) when the shaft portion (218) is everted.

## Patentansprüche

1. Anker (110), umfassend;
einen Basisabschnitt (214), der eine im Allgemeinen rohrförmige Form aufweist, die ein Lumen definiert, das eine Mittelachse (213) aufweist, wobei das Basisabschnitt (214) einen Eingriffsabschnitt (216) umfasst, der konfiguriert ist, damit der Basisabschnitt (214) in ein Zuführsystem eingreift; und
eine Vielzahl von Ankerarmen mit Formgedächtnis (212), die mit dem Basisabschnitt (214) gekoppelt sind, sodass die Vielzahl der Ankerarme mit Formgedächtnis (212) konfiguriert sind, um im Wesentlichen parallel zu einer Mittelachse (213) des Basisabschnitts (214) vor dem Einsatz zu sein, wobei jede der Vielzahl der Ankerarme mit Formgedächtnis (212), die
ein Gewebe durchdringenden Punkt (219) umfasst, der an und durch einen Schaftabschnitt (218) gekoppelt und getragen wird, der zum Umstülpen während des Einsatzes konfiguriert ist, und
wobei jeder der Schaftabschnitte konfiguriert ist, von der Mittelachse (213) des Basisabschnitts weg während des Einsatzes umzustülpen; und
zumindest ein Flanschelement (217), das ein oder mehrere Flanschelementarme (1400) umfasst, die einen Abschnitt von dem einen oder mehreren Flanschelementarmen (1400) aufweisen, der sich im Wesentlichen in eine Position von ungefähr 90 Grad von der Mittelachse (213) des Basisabschnitts (214) des Ankers (110) umstülpt; und
wobei das zumindest eine Flanschelement (217) atraumatisch ist; **gekennzeichnet durch** ein fluoriertes Polymer-Biomaterial, das an dem Flanschelement (217) als eine Kappe (1500) angebracht ist.

2. Anker (110) nach Anspruch 1, wobei der Anker (110) entlang eines länglichen Elements (104) einsetzbar ist.

3. Anker (110) nach Anspruch 2, wobei der Eingriffsabschnitt (216) ein Gewindeabschnitt ist, das durch das längliche Element (104) in Eingriff gebracht werden kann, um die Vielzahl der Ankerarme mit Formgedächtnis (212) aus einem Gewebe einzuziehen.

4. Anker (110) nach Anspruch 3, wobei sich der Gewindeabschnitt entlang einem Innendurchmesser des Basisabschnitts (214) befindet.

5. Anker (110) nach Anspruch 1, wobei der Eingriffsabschnitt (216) einen magnetischen Abschnitt umfasst, der mit einem entsprechenden magnetischen Abschnitt eines Zuführsystem in Eingriff gebracht werden kann, um den Anker (110) einzusetzen.

6. Anker (110) nach Anspruch 1, wobei der Gewebe durchdringende Punkt (219) von jeder der Vielzahl von Ankerarmen mit Formgedächtnis (212) im Wesentlichen parallel zu der Mittelachse (213) des Basisabschnitts (214) zeigt, wenn der Schaftabschnitt (218) umgestülpt wird.

## Revendications

1. Dispositif d'ancrage (110), comprenant :
une partie base (214) possédant une forme généralement tubulaire définissant une lumière dotée d'un axe central (213), ladite partie base (214) comprenant une partie de mise en prise (216) conçue pour mettre en prise temporairement ladite partie base (214) avec un système d'administration ; et
une pluralité de bras de dispositif d'ancrage à mémoire de forme (212) couplée à la partie base (214) de sorte que la pluralité de bras de dispositif d'ancrage à mémoire de forme (212) soient conçus pour être sensiblement parallèle à un axe central (213) de la partie base (214) avant son déploiement, chacun de la pluralité de bras de dispositif d'ancrage à mémoire de forme (212)
comprenant un point de pénétration dans le tissu (219) couplé à une partie de tige (218) et soutenu par celle-ci qui est configurée pour s'évaser pendant le déploiement, et
chacune desdites parties de tige étant conçue pour s'évaser depuis l'axe central (213) de la partie base pendant le déploiement ; et
au moins un élément de bride (217) comprenant un ou plusieurs bras d'élément de bride (1400) comportant une partie dudit ou desdits bras d'élément de bride (1400) s'évasant sensiblement jusqu'à une position faisant un angle d'environ 90 degrés par rapport à l'axe central (213) de la partie base (214) du dispositif d'ancrage (110) ; et
le au moins un élément de bride (217) étant atraumatique ; **caractérisé par** un biomatériau de polymère fluoré attaché à l'élément de bride (217) comme un bouchon (1500).

2. Dispositif d'ancrage (110) selon la revendication 1, ledit dispositif d'ancrage (110) étant déployé le long d'un élément allongé (104).

3. Dispositif d'ancrage (110) selon la revendication 2, ladite partie de mise en prise (216) étant une partie filetée pouvant venir en prise avec ledit élément allongé (104) afin de rétracter la pluralité de bras de dispositif d'ancrage à mémoire de forme (212) d'un tissu.

4. Dispositif d'ancrage (110) selon la revendication 3, ladite partie filetée étant située le long d'un diamètre interne de la partie base (214).

5. Dispositif d'ancrage (110) selon la revendication 1, ladite partie de mise en prise (216) comprenant une partie magnétique pouvant venir en prise avec une partie magnétique correspondante d'un système d'administration afin de déployer ledit dispositif d'ancrage (110).

6. Dispositif d'ancrage (110) selon la revendication 1, ledit point de pénétration dans le tissu (219) de chacun de la pluralité de bras de dispositif d'ancrage à mémoire de forme (212) pointant sensiblement selon une direction parallèle à l'axe central (213) de la partie base (214) lorsque la partie de tige (218) est évasée.
